# FASCICULE DE BREVET EUROPEEN

(11) **EP 0 844 245 B1**
(45) Date de publication et mention de la délivrance du brevet: **09.05.2001**
(21) Numéro de dépôt: 97402821.9
(22) Date de dépôt: 24.11.1997
(51) Int. Cl.: C07D 311/66, C07D 311/72, A61K 31/355, C07D 405/12, A61K 31/35

(54) **Dérivés du benzopyrane, leur procédé de préparation et les compositions pharmaceutiques qui les contiennent**
Benzopyranderivate, Verfahren zu deren Herstellung und diese enthaltende pharmazeutische Zusammenstellungen
Benzopyran derivatives, process for their preparation and pharmaceutical compositions containing them

(30) Priorité: 26.11.1996 FR 9614470
(43) Date de publication de la demande: 27.05.1998
(73) Titulaire: ADIR ET COMPAGNIE, 92415 Courbevoie Cédex (FR)
(72) Inventeur: Muller, Timothée, 44450 La Chapelle Basse Mer (FR); Moulin, Claudie, 35740 Pace (FR); Duflos, Muriel, La Croix Rouge, 44640 Vue (FR); Robert-Piessard, Sylvie, 44200 Nantes (FR); Le Baut, Guillaume, 44230 Saint Sebastien Sur Loire (FR); Tonnerre, Alain, La Croix Jeannette, 44340 Bouguenais (FR); Caignard, Daniel-Henri, 78230 Le Pecq (FR); Manechez, Dominique, 59290 Wasquehal (FR); Renard, Pierre, 78000 Versailles (FR)

(56) Documents cités:
- EP-A- 0 512 899
- WO-A-95/33742

## Description

La présente invention concerne de nouveaux dérivés benzopyraniques, leur procédé de . préparation et les compositions pharmaceutiques qui les contiennent.

Plus spécifiquement, l'invention concerne des N-sulfonyl-benzopyrane-2-carboxamides et leurs dérivés, famille qui n'a jamais été décrite à notre connaissance à ce jour.

La demanderesse a découvert ces composés comme particulièrement intéressants en thérapeutique dans le traitement du diabète ainsi que dans celui des effets secondaires de la maladie diabétique.

La demande EP A 0 512 899 qui constitue l'art antérieur le plus proche décrit des composés benzopyranniques qui possèdent une activité antioxydante. Plus spécifiquement, la présente invention concerne des composés de formule générale (I) : 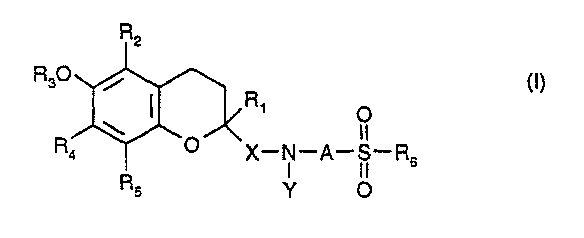 dans laquelle :
- R₁: représente un groupement alkyle (C₁-C₉) linéaire ou ramifié ;
- R₂, R₄, R₅: identiques ou différents, représentent chacun indépendamment l'un de l'autre, un atome d'hydrogène ou un groupement alkyle (C₁-C₉) linéaire ou ramifié ;
- R₃: représente un atome d'hydrogène, un groupement alkyle (C₁-C₉) linéaire ou ramifié, un groupement acyle (C₁-C₉) linéaire ou ramifié, un groupement carboxyalkyle (C₁-C₉) linéaire ou ramifié, un groupement alkoxycarbonyle (C₁-C₉) linéaire ou ramifié, ou un groupement 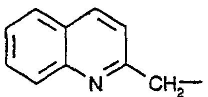 éventuellement substitué par un halogène en position 7 ;
- X: représente un groupement carbonyle ou un groupement méthylène ;
- Y: représente un atome d'hydrogène, un groupement alkyle (C₁-C₉) linéaire ou ramifié, ou un groupement aryle éventuellement substitué ;
- A: représente
- soit une liaison simple et alors :
   R6 représente un groupement alkyle (C₁-C₉) linéaire ou ramifié (éventuellement substitué par un groupement aryle lui-même éventuellement substitué), ou un groupement aryle éventuellement substitué ;
- soit un groupement alkylphényle (éventuellement substitué sur le phényle) -R_{b}-Ph-, la partie R_{b} alkyle (C₁ - C₉) linéaire ou ramifié étant rattachée au groupement 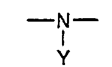 et alors :
   R₆ représente :
   - un groupement isocyanate,
   - un groupement amino éventuellement substitué par un ou deux groupements alkyles (C₁-C₉) linéaires ou ramifiés, identiques ou différents, ou par un groupement alcoxycarbonyle (C₁-C₉) linéaire ou ramifié,
   - ou un groupement urée substitué -NH-CO-NH-R₇ avec R₇ représentant un groupement phényle (éventuellement substitué), un groupement cycloalkyle (C₅-C₈) (éventuellement substitué par un groupement alkyle (C₁-C₉) linéaire ou ramifié), ou un groupement 3-azabicyclo [3.3.0]octyle,
leurs isomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, tartrique, maléique, fumarique, oxalique, méthane sulfonique, camphorique, éthane sulfonique, citrique, phosphonique, acétique, trifluoroacétique, lactique, pyruvique, malonique, succinique, glutarique, ascorbique, etc...

Parmi les bases pharmaceutiquement acceptables, on peut citer à titre non limitatif les hydroxydes de sodium, de potassium, de calcium, les carbonates de sodium, de potassium, de calcium, la triéthylamine, la diéthylamine, l'éthanolamine, etc...

Par groupement aryle on entend un groupement phényle ou naphtyle et par groupement aryle substitué ou phényle substitué on entend une substitution par 1 à 3 groupements choisis identiques ou différents et indépendamment l'un de l'autre parmi :
- alkyle (C₁-C₉) linéaire ou ramifié,
- alkoxy (C₁-C₉) linéaire ou ramifié,
- alkoxycarbonyle (C₁-C₉) linéaire ou ramifié,
- carboxyle,
- carboxyalkyle (C₁-C₉) linéaire ou ramifié,
- acyle (C₁-C₉) linéaire ou ramifié,
- alkylcarbonylamino (C₁-C₉) linéaire ou ramifié,
- aminoalkyle (C₁-C₉) linéaire ou ramifié,
- 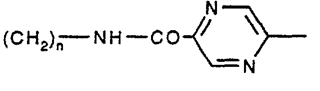 où n est compris entre 1 et 6 inclus,
- amino éventuellement substitué par un ou deux groupements alkyles (C₁ - C₉) linéaires ou ramifiés, identiques ou différents,
- alkényle (C₁-C₉) linéaire ou ramifié,
- nitro,
- halogéno,
- trihalogénométhyle,
- phtalimidoalkyle (C₁-C₉) linéaire ou ramifié.

Les composés préférés selon l'invention sont ceux pour lesquels :
- R₁, R₂, R₄ et R₅,: identiques ou différents, représentent un groupement alkyle (C₁-C₉) linéaire ou ramifié,
- R₃: représente un atome d'hydrogène ou un groupement acyle (C₁-C₉) linéaire ou ramifié,
- X: représente un groupement carbonyle,
- Y: représente un atome d'hydrogène ou un groupement alkyle (C₁-C₉) linéaire ou ramifié et de façon préférentielle un atome d'hydrogène,
- A: représente
- une liaison simple et alors R6 représente avantageusement un groupement aryle éventuellement substitué,
- ou un phényléthyle et alors R₆ représente avantageusement une urée substituée -NH-CO-NH-R₇ dans laquelle R₇ est tel que défini précédemment,
et le cas échéant, leur isomères ainsi que leurs sels d'addition à un acide ou à une base pharmaceutiquement acceptable.

D'une façon particulièrement avantageuse, les composés préférés selon l'invention sont ceux pour lesquels A représente une liaison simple et R₆ représente un groupement phényle substitué par un groupement alkyle (C₁-C₉) linéaire ou ramifié, un halogène ou un groupement 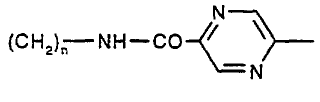 où n est compris entre 1 et 6 inclus.

Très avantageusement, les composés préférés de l'invention sont le 4-Chloro, le 4-Bromo et le 4 Méthyl-N-(6-acétoxy-3,4-dihydro-2,5,7,8-tétraméthyl-2H-1-benzopyrane-2-carbonyl)-benzènesulfonamide, leurs isomères ainsi que leurs sels d'addition à un acide ou à une base pharmaceutiquement acceptable.

L'invention s'étend également au procédé de préparation des composés de formule (I) caractérisé en ce que l'on utilise comme produit de départ, un composé de formule (II) : 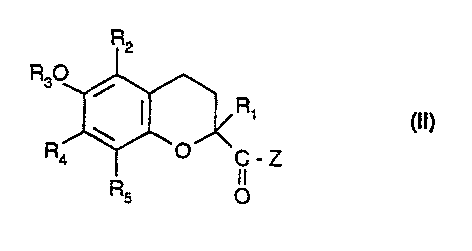 dans laquelle :
Z représente un groupement hydroxyle ou un atome d'halogène, et R₁, R₂, R₃, R₄ et R₅ ont la même signification que dans la formule (I),
que l'on condense :
- soit, lorsque A dans la formule (I) représente une liaison simple, avec un composé de formule (III) : 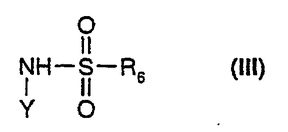 dans laquelle Y et R₆ ont la même signification que dans la formule (I),
   pour conduire au composé de formule (I/a), cas particulier des composés de formule (I) : 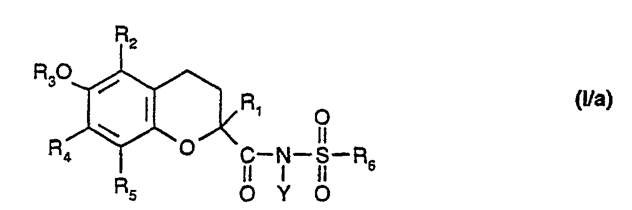 dans laquelle R₁, R₂, R₃, R₄, R₅, R₆ et Y ont la même signification que dans la formule (I), composé de formule (I/a) dont on réduit la fonction carbonyle, si on le souhaite, pour conduire au composé de formule (l/b), cas particulier des composés de formule (I) : 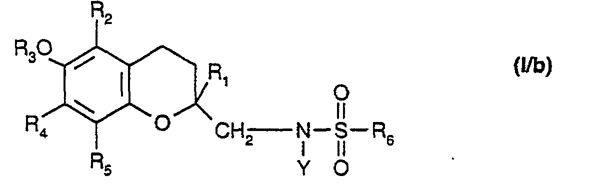 dans laquelle R₁, R₂, R₃, R₄, R₅, R₆, et Y ont la même signification que précédemment (I),
- soit, lorsque A dans la formule (I) représente un groupement alkylphényle -R_{b}-Ph-, avec un composé de formule (IV) : 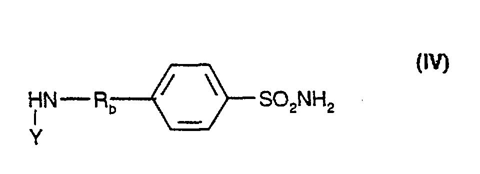 dans laquelle Y et R_{b} ont la même signification que dans la formule (I), pour conduire à un mélange de deux composés de formules respectives (I/c) et (I/d), cas particulier des composés de formule (I) 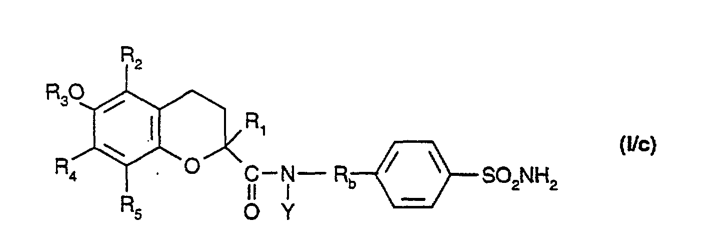 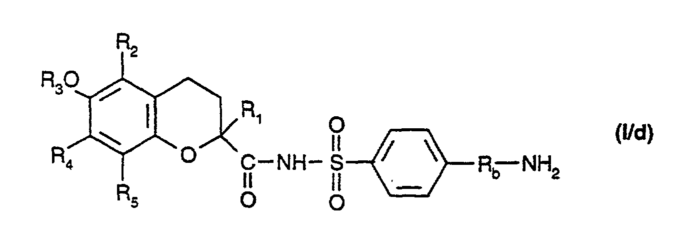 formules (I/c) et (I/d) dans lesquelles R_{b}, R₁, R₂, R₃, R₄, R₅ et Y ont la même signification que dans la formule (I),
   mélange dont on isole selon les procédés classiques de chromatographie, le composé de formule (I/c) que l'on traite par le phosgène pour conduire au composé de formule (I/e), cas particulier des composés de formule (I) : 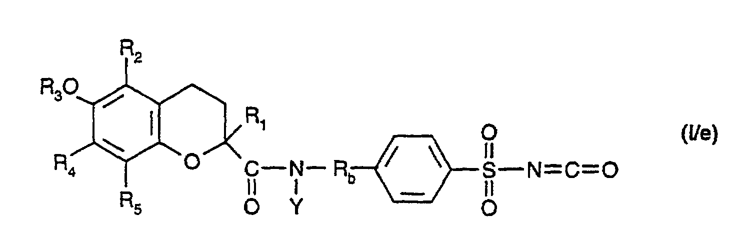 dans laquelle, R_{b}, R₁, R₂, R₃, R₄, R₅ et Y ont la même signification que dans la formule (I), composé de formule (I/e) que l'on traite par un composé de formule (V) :

   H₂N - R₇ (V)

   dans laquelle R₇ a la même signification que dans la définition générale de la formule (I), pour donner des composés de formule (l/f), cas particulier des composés de formule (I) 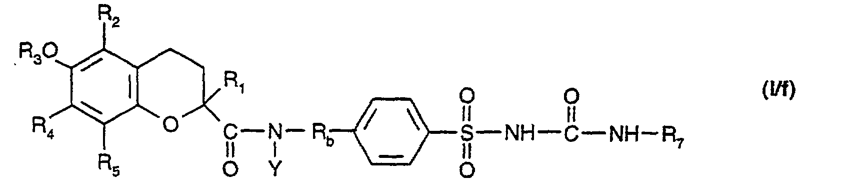 dans laquelle R_{b}, R₁, R₂, R₃, R₄, R₅, R₇ et Y ont la même signification que celle présentée dans la description générale de la formule (I),
composés de formules (I/a) à (I/f), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les composés de formule (I) possèdent des propriétés pharmacologiques intéressantes.

En particulier ils ont la propriété de s'opposer à l'augmentation de la glycémie chez l'animal rendu artificiellement diabétique après une charge en glucose et à ce titre ils sont intéressants dans le traitement de la maladie diabétique.

De plus ils ont révélé de bonnes propriétés antioxydantes et à ce titre sont intéressants dans le traitement des complications de la maladie diabétique dues à des micro ou macro angiopathies qui entraînent néphropathie, neuropathie et rétinopathies.

Les composés de l'invention sont en outre peu toxiques.

La présente invention a également pour objet les compositions pharmaceutiques renfermant comme principe actif au moins un composé de formule générale (I) ou un de ses sels d'addition à un acide pharmaceutiquement acceptable ou le cas échéant à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublingaux, les sachets, paquets, les gélules, glossettes, tablettes, suppositoires, crèmes, pommades, gels dermiques, les préparations injectables, les suspensions buvables, etc...

La posologie varie selon l'âge et le poids du patient, la nature et la sévérité de l'affection ainsi que la voie d'administration. Celle-ci peut être orale, nasale, rectale ou parentérale.

D'une manière générale, la posologie unitaire s'échelonne entre 0,05 mg et 500 mg de une à trois prises par 24 heures.

Les exemples suivants illustrent l'invention mais ne la limitent en aucune façon.
Les produits de départ utilisés sont des produits connus ou préparés selon des modes opératoires connus.

Les spectres de résonance magnétique nucléaire ¹H ont été réalisés en utilisant le TMS (tétraméthylsilane) comme référence interne. Les déplacements chimiques sont exprimés en partie par million (p.p.m). Les spectres infrarouge ont été effectués sous forme de pastille de bromure de potassium renfermant environ 1 % du produit à analyser.

### EXEMPLE 1 :

### N-(6-acétoxy-3,4-dihydro-2,5,7,8-tétraméthyl-2H-1-benzopyrane-2-carbonyl)-méthanesulfonamide

### Méthode 1 :

Dissoudre un mélange de 1,5 g de Trolox acétylé ou acide 6-acétoxy-3,4-dihydro-2,5,7,8-tétraméthyl-2H-1-benzopyrane-2-carboxylique (5,13 mmol), de 0,52 g (5,4 mmol) de méthanesulfonamide, de 0,66 g (5,4 mmol) de 4-diméthylaminopyridine et de 0,84 ml (5,4 mmol) de N, N'-diisopropylcarbodiimide dans 90 ml de dichlorométhane anhydre ; agiter à température ambiante durant 18 heures.

Filtrer l'urée qui a partiellement précipité. Concentrer le filtrat, laisser refroidir et filtrer de nouveau l'urée. Recommencer l'opération une à deux fois. Verser le dernier filtrat sur une solution d'acide chlorhydrique 1M, extraire la phase aqueuse par du dichlorométhane, laver la phase organique par une solution saturée de NaCI. Sécher sur sulfate de sodium anhydre et évaporer le solvant. Précipiter le produit dans l'éther diisopropylique, filtrer et laver à l'éthanol pour éliminer les traces d'urée. Recristalliser dans l'éther diisopropylique pour recueillir 1,38 g d'une poudre blanche.
Rendement : 73 %
Point de fusion : 188°C (éther diisopropylique)
C₁₇H₂₃NO₆S
Mr : 369,44
IR (KBr), ν cm⁻¹ : 3360 (νNH) ; 1735 (νC=O ester) ; 1715 (νC=O amide) ; 1340 (νSO₂s) ; 1220 (com NH /CN) ; 1170 (νSO₂as).

### EXEMPLE 2 :

### 4-Fluoro-N-(6-acétoxy-3,4-dihydro-2,5,7,8-tétraméthyl-2H-1-benzopyrane-2-carbonyl)-benzènesulfonamide

On procède comme dans la méthode 1 en utilisant comme réactif le 4-Fluoro-benzènesulfonamide à la place du méthanesulfonamide.
Point de fusion : 130°C

### EXEMPLE 3 :

### 4-Chloro-N-(6-acétoxy-3,4-dihydro-2,5,7,8-tétraméthyl-2H-1-benzopyrane-2-carbonyl)-benzènesulfonamide

On procède comme dans la méthode 1 en utilisant comme réactif le 4-Chloro-benzènesulfonamide à la place du méthanesulfonamide.
Point de fusion : 173°C

### EXEMPLE 4 :

### 4-Bromo-N-(6-acétoxy-3,4-dihydro-2,5,7,8-tétraméthyl-2H-1-benzopyrane-2-carbonyl)-benzènesulfonamide

On procède comme dans la méthode 1 en utilisant comme réactif le 4-Bromo-benzènesulfonamide à la place du méthanesulfonamide.
Point de fusion : 153-155°C

### EXEMPLE 5 :

### 2-Méthyl-N-(6-acétoxy-3,4-dihydro-2,5,7,8-tétraméthyl-2H-1-benzopyrane-2-carbonyl)-benzènesulfonamide

On procède comme dans la méthode 1 en utilisant comme réactif le 2-Méthyl-benzènesulfonamide à la place du méthanesulfonamide.
Point de fusion : 165-167°C

### EXEMPLE 6 :

### N-(6-acétoxy-3,4-dihydro-2,5,7,8-tétraméthyl-2H-1-benzopyrane-2-carbonyl)-benzylsufonamide

On procède comme dans la méthode 1 en utilisant comme réactif le benzylsufonamide à la place du méthanesulfonamide.
Point de fusion : 150°C

### EXEMPLE 7 :

### 4-Méthoxycarbonyl-N-(6-acétoxy-3,4-dihydro-2,5,7,8-tétraméthyl-2H-1-benzopyrane-2-carbonyl)-benzènesulfonamide

On procède comme dans la méthode 1 en utilisant comme réactif le 4-Méthoxycarbonyl-benzènesulfonamide à la place du méthanesulfonamide.
Point de fusion : 185°C

### EXEMPLE 8 :

### N-(6-acétoxy-3,4-dihydro-2,5,7,8-tétraméthyl-2H-1-benzopyrane-2-carbonyl)-2-naphtalènesulfonamide

On procède comme dans la méthode 1 en utilisant comme réactif le 2-naphtalènesulfonamide à la place du méthanesulfonamide.
Point de fusion : 183°C

### EXEMPLE 9 :

### N-(6-acétoxy-3,4-dihydro-2,5,7,8-tétraméthyl-2H-1-benzopyrane-2-carbonyl)-1-naphtalènesulfonamide

On procède comme dans la méthode 1 en utilisant comme réactif le 1-naphtalènesulfonamide à la place du méthanesulfonamide.
Point de fusion : 167-169°C

### EXEMPLE 10:

### 4-Isopropyl-N-(6-acétoxy-3,4-dihydro-2,5,7,8-tétraméthyl-2H-1-benzopyrane-2-carbonyl)-benzènesulfonamide

### Méthode 2 :

Additionner goutte à goutte une solution de 2 g (6,84 mmol) de Trolox acétylé dans 17 ml de tétrahydrofurane anhydre à une solution agitée de 1,1 g (6,85 mmol) de carbonyl diimidazole dans 13 ml de tétrahydrofurane anhydre, sous azote. Agiter trente minutes à température ambiante, puis porter à reflux trente minutes. Laisser revenir à température ambiante. Ajouter 1,36 g (6,85 mmol) de 4-isopropylbenzènesulfonamide et agiter dix minutes. Rajouter goutte à goutte une solution de 1,03 ml (6,85 mmol) de DBU ou 1,8 diazabicyclo [5.4.0]undec-7-ène dans 6 ml de THF anhydre. Agiter une nuit à température ambiante. Evaporer le solvant. Reprendre le résidu au dichlorométhane, laver par une solution aqueuse 1 M d'acide chlorhydrique, puis par une solution aqueuse saturée de chlorure de sodium. Sécher sur sulfaté de sodium anhydre. Evaporer le solvant. Purifier l'huile obtenue par chromatographie sur gel de silice en éluant par un mélange dichlorométhane : éthanol (99 : 1). Recristalliser dans l'éther diisopropylique 2,61 g d'une poudre blanche.
Rendement : 78 %
Point de fusion : 124-126°C (éther diisopropylique)
C₂₅H₃₁NO₆S
Mr : 489,59
IR (KBr), v cm⁻¹ : 3346 (νNH) ; 1766 (νC=O ester) ; 1726 (νC=O amide) ; 1597 (δNH) ; 1338 (νSO₂s) ; 1204 (comb NH / CN ; 1081 (νSO₂as)

### EXEMPLE 11 :

### 4-Ethyl-N-(6-acétoxy-3,4-dihydro-2,5,7,8-tétraméthyl-2H-1-benzopyrane-2-carbonyl)-benzènesulfonamide

On procède comme dans la méthode 2 en utilisant comme réactif le 4-éthyl-benzènesulfonamide.
Point de fusion : 130°C

### EXEMPLE 12 :

### 3-Méthyl-N-(6-acétoxy-3,4-dihydro-2,5,7,8-tétraméthyl-2H-1-benzopyrane-2-carbonyl)-benzènesulfonamide

On procède comme dans la méthode 2 en utilisant comme réactif le 3-Méthyl-benzènesulfonamide.
Point de fusion : 89-91°C

### EXEMPLE 13 :

### 2-Nitro-N-(6-acétoxy-3,4-dihydro-2,5,7,8-tétraméthyl-2H-1-benzopyrane-2-carbonyl)-benzènesulfonamide

On procède comme dans la méthode 2 en utilisant comme réactif le 2-Nitro-benzènesulfonamide.
Point de fusion : 170°C

### EXEMPLE 14 :

### 4-Acétamido-N-(6-acétoxy-3,4-dihydro-2,5,7,8-tétraméthyl-2H-1-benzopyrane-2-carbonyl)-benzènesulfonamide

On procède comme dans la méthode 1 en utilisant comme réactif le 4-Acétamido-benzènesulfonamide.
Point de fusion : 182-184°C

### EXEMPLE 15 :

### 4-Méthyl-N-(6-acétoxy-2,5,7,8-tétraméthyl-3,4-dihydro-2H-1-benzopyrane-2-carbonyl)-benzénesulfonamide

### Méthode 3

A une solution de 3 g (10,25 mmol) de Trolox acétylé, dans 31 ml de toluène anhydre, ajouter 1,13 ml de chlorure de thionyle et trois gouttes de diméthylformamide. Chauffer au reflux pendant trois heures. Laisser refroidir, puis évaporer sous vide le solvant et l'excès de chlorure de thionyle. Reprendre le chlorure d'acide ainsi obtenu par 10 ml de THF anhydre.

Additionner lentement 1,76 g (10,25 mmol) de p-toluènesulfonamide à une solution de 0,82 g (20,50 mmol) d'hydrure de sodium à 60 % (préalablement lavé avec du toluène anhydre) dans 15 ml de THF anhydre. Sur ce mélange, verser progressivement la solution de chlorure d'acide. Agiter à température ambiante pendant 24 heures. Détruire l'excès d'hydrure de sodium par une solution aqueuse saturée d'hydrogénocarbonate de sodium. Laver la phase aqueuse au dichlorométhane, puis l'acidifier par une solution aqueuse 1M d'acide chlorhydrique. Extraire au dichlorométhane, sécher sur sulfate de sodium anhydre. Après élimination du solvant, purifier l'huile brune obtenue par chromatographie sur gel de silice en éluant par un mélange dichlorométhane : éthanol (99 : 1). Recristalliser dans l'éther diisopropylique et recueillir 3,55 g d'une poudre blanche.
Rendement : 78 %
F°C : 127 (éther diisopropylique)
C₂₃H₇NO₆S
Mr : 445,54

### EXEMPLE 16 :

### 4-tert-Butyl-N-(6-acétoxy-2,5,7,8-tétraméthyl-3,4-dihydro-2H-1-benzopyrane-2-carbonyl)-benzènesulfonamide

On procède comme dans la méthode 3 en utilisant comme réactif le 4-tert-Butyl-benzènesulfonamide à la place du para-toluènesulfonamide
Point de fusion : 125°C

### EXEMPLE 17 :

### 4-Méthyl-N,N-(méthyl)(6-acétoxy-3,4-dihydro-2,5,7,8-tétraméthyl-2H-1-benzopyrane-2-carbonyl)-benzènesulfonamide

On procède comme dans la méthode 3 en utilisant comme réactif le N-(méthyl)-paratoluènesulfonamide à la place du para-toluènesulfonamide.
Point de fusion : 132°C

### EXEMPLE 18 :

### N-(3,4-dihydro-6-hydroxy-2,5,7,8-tétraméthyl-2H-1benzopyrane-2-carbonyl)-méthanesulfonamide

### Méthode 4

A une solution de 1 g (2,7 mmol) du dérivé de l'exemple 1 dans 15 ml d'éthanol, ajouter 6,1 équivalents d'une solution aqueuse d'hydroxyde de sodium 2M (16,5 mmol). Agiter sous courant d'azote durant deux heures. Diluer le mélange par de l'eau, acidifier par une solution aqueuse d'acide acétique 1M. Extraire au dichlorométhane. Laver la phase organique par de l'eau. Sécher sur sulfate de sodium anhydre. Evaporer le solvant, puis recristalliser dans l'éther diisopropylique pour recueillir 0,71 g d'une poudre blanche.
Rendement : 80 %
F°C : 185 (éther diisopropylique)
C₁₅H₂₁NO₅S
Mr : 327,40
IR (KBr) , ν cm⁻¹ : 3420 (νOH) ; 3375 (νNH) ; 1700 (νC=O amide) ; 1330 (νSO₂s) ; 1250 (comb NH / CN) ; 1175 (νSO₂as)

### EXEMPLE 19 :

### 4-Fluoro-N-(3,4-dihydro-6-hydroxy-2,5,7,8-tétraméthyl-2H-1-benzopyrane-2-carbonyl)-benzènesulfonamide

On procède comme dans la méthode 4 en utilisant comme substrat de départ le composé de l'exemple 2.
Point de fusion : 139°C

### EXEMPLE 20 :

### 4-Chloro-N-(3,4-dihydro-6-hydroxy-2,5,7,8-tétraméthyl-2H-1-benzopyrane-2-carbonyl)-benzènesulfonamide

On procède comme dans la méthode 4 en utilisant comme substrat de départ le composé de l'exemple 3.
Point de fusion : 110°C

### EXEMPLE 21 :

### 4-Bromo-N-(3,4-dihydro-6-hydroxy-2,5,7,8-tétraméthyl-2H-1-benzopyrane-2-carbonyl)-benzènesulfonamide

On procède comme dans la méthode 4 en utilisant comme substrat de départ le composé de l'exemple 4.
Point de fusion : 115-117°C

### EXEMPLE 22 :

### 4-Méthyl-N-(3,4-dihydro-6-hydroxy-2,5,7,8-tétraméthyl-2H-1-benzopyrane-2-carbonyl)-benzènesulfonamide

On procède comme dans la méthode 4 en utilisant comme substrat de départ le composé de l'exemple 15.
Point de fusion : 95°C

### EXEMPLE 23 :

### 4-tert-Butyl-N-(3,4-dihydro-6-hydroxy-2,5,7,8-tétraméthyl-2H-1-benzopyrane-2-carbonyl)-benzènesulfonamide

On procède comme dans la méthode 4 en utilisant comme substrat de départ le composé de l'exemple 16.
Point de fusion : 122-124°C

### EXEMPLE 24 :

### N-(3,4-dihydro-6-hydroxy-2,5,7,8-tétraméthyl-2H-1-benzopyrane-2-carbonyl)-benzylsufonamide

On procède comme dans la méthode 4 en utilisant comme substrat de départ le composé de l'exemple 6.
Point de fusion : huile

### EXEMPLE 25 :

### Acide-N-(3,4-dihydro-6-hydroxy-2,5,7,8-tétraméthyl-2H-1-benzopyrane-2-carbonyl)-benzoïque-sulfonamide

On procède comme dans la méthode 4 en utilisant comme substrat de départ le composé de l'exemple 7.
Point de fusion : 115-117°C

### EXEMPLE 26 :

### 2-Nitro-N-(3,4-dihydro-6-hydroxy-2,5,7,8-tétraméthyl-2H-1-benzopyrane-2-carbonyl)-benzènesulfonamide

On procède comme dans la méthode 4 en utilisant comme substrat de départ le composé de l'exemple 13.
Point de fusion : 167-169°C

### EXEMPLE 27 :

### 4-Fluoro-N- (3,4-dihydro-6-hydroxy-2,5,7,8-tétraméthyl-2H-1-benzopyrane-2-ylméthyl)-benzènesulfonamide

### Méthode 5

Dissoudre 2 g (4,45 mmol) de l'exemple 2 dans 150 ml d'éther diéthylique anhydre. Ajouter lentement 0,84 g (22,25 mmol) d'aluminohydrure de lithium, puis porter le mélange à reflux pendant 4 heures. Après refroidissement, détruire l'excès d'hydrure par addition lente d'eau glacée. Filtrer. Extraire le filtrat par du dichlorométhane, sécher la phase organique et évaporer le solvant. Purifier le produit sur colonne de silice en éluant par un mélange dichlorométhane : éthanol (99 : 1). Recristalliser le produit obtenu dans l'éther diisopropylique. Récupérer 1,13 g d'une poudre blanche.
Rendement : 65 %
F°C : 136-138°C(étherdiisopropylique)
C₂₀H₂₄FNO₄S
Mr : 393,48
IR (KBr), ν cm⁻¹ : 3450 (νOH) ; 3270 (νNH) ; 1580 (δNH) 1325 (ν SO₂s) ; 1140 (νSO₂as)

### EXEMPLE 28 :

### 4-Méthyl-N-(3,4-dihydro-6-hydroxy-2,5,7,8-tétraméthyl-2H-1-benzopyrane-2-ylméthyl)-benzènesulfonamide

On procède comme dans la méthode 5 en utilisant comme substrat de départ le composé de l'exemple 15.
Point de fusion : 152-154°C

### EXEMPLE 29 :

### 4-Chloro-N-(3,4-dihydro-6-hydroxy-2,5,7,8-tétraméthyl-2H-1-benzopyrane-2-ylméthyl)-benzènesulfonamide

On procède comme dans la méthode 5 en utilisant comme substrat de départ le composé de l'exemple 3.
Point de fusion : 135-137°C

### EXEMPLE 30 :

### 4-Bromo-N-(3,4-dihydro-6-hydroxy-2,5,7,8-tétraméthyl-2H-1-benzopyrane-2-ylméthyl)-benzènesulfonamide

On procède comme dans la méthode 5 en utilisant comme substrat de départ le composé de l'exemple 4.
Point de fusion : 161°C

### EXEMPLE 31 :

### N-(3,4-dihydro-6-hydroxy-2,5,7,8-tétraméthyl-2H-1-benzopyrane-2-ylméthyl)-méthanesulfonamide

On procède comme dans la méthode 5 en utilisant comme substrat de départ le composé de l'exemple 1.
Point de fusion : 125°C

### EXEMPLE 32 :

### 4-Bromo-N-(6-acétoxy-3,4-dihydro-2,5,7,8-tétraméthyl-2H-1-benzopyrane-2-ylméthyl)-benzènesulfonamide

### Méthode 6

Dissoudre 10 g (39,95 mmol) du dérivé obtenu dans l'exemple 30 dans 30 ml de pyridine anhydre, refroidir la solution à l'aide d'un bain de glace, puis ajouter goutte à goutte 18,8 ml d'anhydre acétique. Agiter pendant 2 heures à température ambiante. Verser le mélange sur de la glace, extraire le produit par du dichlorométhane. Laver la phase organique par une solution aqueuse d'acide chlorhydrique 1M, puis à l'eau. Sécher sur sulfate de sodium anhydre. Après évaporation du solvant, recueillir un résidu solide, le recristalliser dans l'éther diisopropylique pour obtenir 11 g d'une poudre blanche.
Rendement: 94 %
Point de fusion 148°C (éther diisopropylique)
C₂₃H₂₉NO₅S
Mr : 431,555
IR (KBr), ν cm⁻¹ : 3304 (νNH) ; 1728 (νC=O) ; 1459 (δNH) 1331 (νSO₂s) 1159 (νSO₂as)

### EXEMPLE 33 :

### 4-Méthyl-N-(6-acétoxy-3,4-dihydro-2,5,7,8-tétraméthyl-2H-1-benzopyran-2-ylméthyl)-benzènesulfonamide

On procède comme dans la méthode 6 en utilisant comme substrat de départ le composé de l'exemple 28.
Point de fusion : 130°C

### EXEMPLES 34 et 35 :

### 6-Acétoxy-3,4-dihydro-2,5,7,8-tétraméthyl-2H-1-benzopyrane-2-carboxylique acide[2-(4-sulfamoylphényl)-éthyl]-amide (34)

### 4-(2-aminoéthyl)-N-(6-acétoxy-3,4-dihydro-2,5,7,8-tétraméthyl-2H-1-benzopyrane-2-carbonyl) benzènesulfonamide (35)

### Méthode 7

Dissoudre 5 g (17,1 mmol) d'acide 6-acétoxy-3,4-dihydro-2,5,7,8-tétraméthyl-2H-1-benzopyran-2-carboxylique dans du THF anhydre, puis ajouter 3,7 g (17,1 mmol) de carbonyl diimidazole. Agiter à température ambiante pendant 1 heure. Ajouter ensuite 3,43 g (17,1 mmol) de 4-(2-aminoéthyl)benzènesulfonamide. Agiter une nuit à température ambiante. Evaporer le solvant, reprendre par du dichlorométhane, laver à l'eau. Sécher sur sulfate de sodium anhydre. Purifier par chromatographie sur gel de silice en éluant par du dichlorométhane progressivement enrichi en éthanol jusqu'à 2,5 %. Recueillir dans l'ordre 3 g du composé de l'exemple 35, puis 7,6 g du composé de l'exemple 34. Recristalliser l'exemple 34 dans l'éther diisopropylique pour obtenir une poudre blanche.

### EXEMPLE 34 :

Rendement : 94 %
Point de fusion : 166°C (éther diisopropylique)
C₂₄H₃₀N₂O₆S
Mr : 474,57
IR (KBr), ν cm⁻¹ : 3420 (νNH) : 1750 (νC=O ester) ; 1660 (νC=O amide) ; 1530 (δNH) ; 1370 (νSO₂s) 1150 (νSO₂s)

### EXEMPLE 35 :

Rendement : 3,7 %
Huile
C₂₄H₃₀N₂O₆S
Mr : 474,57
IR (film), ν cm⁻¹ : 3440 (νNH) : 1750 (νC=O ester) ; 1690 (νC=O amide) ; 1530 (δNH) ; 1370 (νSO₂S); 1150 (νSO₂s)

### EXEMPLE 36 :

### 6-Acétoxy-3,4-dihydro-2,5,7,8-tétraméthyl-2H-1-benzopyran-2-carboxylique acide [2-(4-isocyanatesulfonyl-phényl)-éthyl]-amide

### Méthode 8

Dans 30 ml de toluène anhydre, dissoudre 1 g (2,10 mmol) de composé obtenu dans l'exemple 34 avec 0,2 ml (2,10 mmol) de chlorure d'oxalyle, puis ajouter 0,4 ml (2,4 mmol) de triéthylamine. Agiter à température ambiante pendant 5 heures. Evaporer le toluène, chromatographier le résidu sur gel de silice neutre en éluant par du dichlorométhane. Recristalliser le produit dans l'éther diisopropylique.
Obtenir ainsi 0,36 g d'un produit blanc cassé.
Rendement : 34 %
F°C : 143
C₂₅H₂₈N₂O₇S
Mr : 500,57
IR (KBr), ν cm⁻¹ : 3430 (νNH) : 1752 (νC=O ester) ; 1675 (νC=O amide) ; 1518 (δNH) ; 1371 (νSO₂ₛ) ; 1174 (νSO₂ₐₛ)

### EXEMPLE 37 :

### 6-Acétoxy-3,4-dihydro-2,5,7,8-tétraméthyl-2H-1-benzopyrane-2-carboxylique acide{2-[4-(N'-[3-azabicyclo[3,3,0]octyl])-uréidosulfonyl-phényl]-éthyl)-amide

### Méthode 9

Dissoudre dans un minimum de dichlorométhane anhydre, 0,36 g (0,70 mmol) de composé obtenu dans l'exemple 36, puis ajouter goutte à goutte 0,1 ml (0,70 mmol) de N-aminoaza-3-bicyclo[3,3,0]octane jusqu'à disparition en c.c.m. de l'isocyanate. Evaporer le solvant, chromatographier le résidu huileux sur gel de silice neutre en éluant par de l'acétate d'éthyle. Obtenir ainsi 0,28 g d'une huile jaune.
Rendement : 62 %
C₃₂H₄₂N₄O₇S
Mr : 626,78
IR (film), ν cm⁻¹ : 3700-3250 (νNH amide et urée) : 1754 (νC=O ester) ; 1666, 1664 (νC=O amide et urée) ; 1524 (δNH); 1369 (νSO₂s) 1163 (νSO₂as)

### EXEMPLE 38 :

### 6-Acétoxy-3,4-dihydro-2,5,7,8-tétraméthyl-2H-1-benzopyrane-2-carboxylique acide{2-[4-(N'-[4-méthylcyclohexyl])-uréidosulfonyl-phényl]-éthyl}-amide

On procède comme dans la méthode 9 en utilisant comme réactif du 4-Méthyl-cyclohexylamine
Rendement : 70 %
Huile
C₃₂H₄₃N₃O₇S
Mr : 613,78
IR (film), ν cm⁻¹ : 3425-3283 (νNH amide et urée) : 1755 (νC=O ester) ; 1668, 1666 (νC=O amide et urée) ; 1524 (δNH) ; 1369 (νSO₂s) ; 1161(νSO₂as)

### EXEMPLE 39 :

### 4-Chloro-N-(6-[(quinoléine-2yl)méthoxy]-3,4-dihydro-2,5,7,8-tétraméthyl-2H-1-benzopyrane-2-carbonyl)-benzènesulfonamide

### Méthode 10

On procède comme dans la méthode 1 en utilisant comme substrat l'acide 6-[(quinoléine-2yl)méthoxy]-3,4-dihydro-2,5,7,8-tétraméthyl-2H-1-benzopyrane-2-carboxylique et le réactif de l'exemple 3.
Point de fusion : 132-134°C

### EXEMPLE 40 :

### 4-Fluoro-N-(6-[(quinoléine-2yl)méthoxy]-3,4-dihydro-2,5,7,8-tétraméthyl-2H-1-benzopyrane-2-carbonyl)-benzènesulfonamide

On procède comme dans la méthode 10 en utilisant le réactif de l'exemple 2 à la place de celui de l'exemple 3.
Point de fusion : 148°C

### EXEMPLE 41 :

### N-(6-[(quinoléine-2yl)méthoxy]-3,4-dihydro-2,5,7,8-tétraméthyl-2H-1-benzopyrane-2-carbonyl)-méthanesulfonamide

On procède comme dans la méthode 10 en utilisant le réactif de l'exemple 1 à la place du réactif de l'exemple 3.
Point de fusion : 135°C

### EXEMPLE 42 :

### 4-Fluoro-N-(6-[(7-chloroquinoléine-2yl)eméthoxy]-3,4-dihydro-2,5,7,8-tétraméthyl-2H-1-benzopyrane-2-carbonyl)-benzénesulfonamide

### Méthode 11

On procède comme dans la méthode 1 en utilisant comme substrat l'acide 6-[(7-chloroquinoléine-2yl)méthoxy]-3,4-dihydro-2,5,7,8-tétraméthyl-2H-1-benzopyrane et le réactif de l'exemple 2.
Point de fusion : 123-125°C

### EXEMPLE 43 :

### 4-Chloro-N-(6-[(7-chloroquinoléine-2yl)méthoxy]-3,4-dihydro-2,5,7,8-tétraméthyl-2H-1-benzopyrane-2-carbonyl)-benzènesulfonamide

On procède comme dans la méthode 11 en utilisant le réactif de l'exemple 3 à la place du réactif de l'exemple 2.
Point de fusion : 141-143°C

### EXEMPLE 44 :

### 4-Bromo-N-(6-méthoxy-3,4-dihydro-2,5,7,8-tétraméthyl-2H-1-benzopyrane-2-carbonyl)-benzènesulfonamide

On procède comme dans la méthode 1 en utilisant comme substrat de départ l'acide 6-méthoxy-3,4-dihydro-2,5,7,8-tétraméthyl-2H-1-benzopyrane-2-carboxylique et le réactif de l'exemple 4.
Point de fusion : 134-136°C

### EXEMPLE 45 :

### 4-Acétamido-N-(6-acétoxy-3,4-dihydro-2,5,7,8-tétraméthyl-2H-1-benzopyrane-2-ylméthyl)-benzènesulfonamide

On procède comme dans la méthode 5 en utilisant comme substrat de départ le composé de l'exemple 14, que l'on traite dans une seconde étape selon la méthode 6.
Point de fusion : 95°C

### EXEMPLE 46 :

### 4-Méthyl-N-(6-acétoxy-3,4-dihydro-2-méthyl-7-tert-butyl-2H-1-benzopyrane-2-carbonyl)-benzènesulfonamide

On procède comme dans la méthode 3 en utilisant comme substrat de départ de l'acide 6-acétoxy-3,4-dihydro-2-méthyl-7-tert-butyl-2H-1benzopyrane-2-carboxylique
Point de fusion : 67°C

### EXEMPLE 47 :

### 4-Méthyl-N-(6-méthoxy-3,4-dihydro-2,5,7,8-tétraméthyl-2H-1-benzopyrane-2-carbonyl)-benzènesulfonamide

On procède comme dans la méthode 3 en utilisant comme substrat de départ de l'acide 6-méthoxy-3,4-dihydro-2,5,7,8-tétraméthyl-2H-1-benzopyrane-2-carboxylique
Point de fusion : 130°C

### EXEMPLE 48 :

### 4-Méthyl-N-(6-isobutyryloxy-3,4-dihydro-2,5,7,8-tétraméthyl-2H-1-benzopyrane-2-carbonyl)-benzènesulfonamide

On procède comme dans la méthode 3 en utilisant comme substrat de départ l'acide 6-isobutyryloxy-3,4-dihydro-2,5,7,8-tétraméthyl-2H-1benzopyrane-2-carboxylique
Point de fusion : 103-104°C

### EXEMPLE 49 :

### 4-Bromo-N-(6-isobutyryloxy-3,4-dihydro-2,5,7,8-tétraméthyl-2H-1-benzopyrane-2-carbonyl)-benzènesulfonamide

On procède comme dans la méthode 1 en utilisant comme substrat de départ celui de l'exemple 48.
Point de fusion : 97-103°C

### EXEMPLE 50 :

### 4-Bromo-N-(6-n-butyryloxy-3,4-dihydro-2,5,7,8-tétraméthyl-2H-1-benzopyrane-2-carbonyl)-benzènesulfonamide

On procède comme dans la méthode 1 en utilisant comme substrat l'acide 6-n-butyryloxy-3,4-dihydro-2,5,7,8-tétraméthyl-2H-1-benzopyrane-2-carboxylique
Point de fusion : 139-142°C

### EXEMPLE 51 :

### 4-Méthyl-N-(6-acétoxy-3,4-dihydro-2,5,7,8-tétraméthyl-2H-1-benzopyrane-2-carbonyl)-benzènesulfonamide

On procède comme dans la méthode 1 à l'exemple 1 en utilisant comme réactif le 4-Méthoxy-benzénesulfonamide
Point de fusion : 144°C

### EXEMPLE 52 :

### 4-Méthyl-N-(6-tert-pentyryloxy-3,4-dihydro-2,5,7,8-tétraméthyl-2H-1-benzopyrane-2-carbonyl)-benzènesulfonamide

On procède comme dans la méthode 3 en utilisant comme substrat de départ l'acide 6-tert-pentyryloxy-3,4-dihydro-2,5,7,8-tétraméthyl-2H-1benzopyrane-2-carboxylique
Point de fusion : 97°C

### EXEMPLE 53 :

### 4-Bromo-N-(6-tert-pentyryloxy-3,4-dihydro-2,5,7,8-tétraméthyl-2H-1-benzopyrane-2-carbonyl)-benzènesulfonamide

On procède comme dans la méthode 1 en utilisant le substrat de l'exemple 52 et le réactif de l'exemple 4.
Point de fusion : 125°C

### EXEMPLE 54 :

### 4-Méthyl-N-(6-nbutyryloxy-3,4-dihydro-2,5,7,8-tétraméthyl-2H-1-benzopyrane-2-carbonyl)-benzènesulfonamide

On procède comme dans la méthode 3 en utilisant le substrat de l'exemple 50.
Point de fusion : 103°C

### EXEMPLE 55 :

### 4-Acétamido-N-(6-tert-pentyryloxy-3,4-dihydro-2,5,7,8-tétraméthyl-2H-1-benzopyrane-2-ylméthyl)-benzènesulfonamide

On procède comme dans la méthode 5 puis comme dans la méthode 6.
Point de fusion : 51°C

### EXEMPLE 56 :

### 4-Méthyl-N-(6-propyryloxy-3,4-dihydro-2,5,7,8-tétraméthyl-2H-1-benzopyrane-2-carbonyl)-benzènesulfonamide

On procède comme dans la méthode 3 en utilisant comme substrat de départ l'acide 6-propyryloxy-3,4-dihydro-2,5,7,8-tétraméthyl-2H-1-benzopyrane-2-carboxylique.
Point de fusion : 98°C

### EXEMPLE 57 :

### 4-Bromo-N-(6-propyryloxy-3,4-dihydro-2,5,7,8-tétraméthyl-2H-1-benzopyrane-2-carbonyl)-benzènesulfonamide

On procède comme dans la méthode 1 en utilisant le substrat de l'exemple 56 et le réactif de l'exemple 4.

### EXEMPLE 58 :

### 6-Hydroxy-3,4-dihydro-2,5,7,8-tétraméthyl-2H-1-benzopyrane-2-carboxylique acide[2-(4-sulfamoylphényl)-éthyl]-amide

On procède comme dans la méthode 4 en utilisant comme substrat le composé de l'exemple 34.
Point de fusion : 184°C

### EXEMPLE 59 :

### N-{4-[2-(5-méthylpyrazine-2-carboxamido)éthyl]benzénesulfonyl)-(6-acétoxy-3,4-dihydro-2,5,7,8-tétraméthyl-2H-1-benzopyrane-2yl)-carboxamide

On procède comme dans la méthode 3 en utilisant comme réactif le 4-[2-(5-méthylpyrazine-2-carboxamido)éthyl]benzènesulfonamide.
Point de fusion : 156-158°C

### EXEMPLE 60 :

### N-{4-[2-(5-méthylpyrazine-2-carboxamido)éthyl]benzénesulfonyl]-(6-propyryloxy-3,4-dihydro-2,5,7,8-tétraméthyl-2H-1-benzopyrane-2-yl)-carboxamide

On procède comme dans la méthode 3 en utilisant le substrat de l'exemple 56 et le réactif de l'exemple 59.
Point de fusion : 112°C

### EXEMPLE 61 :

### N-{4-[2-(5-méthylpyrazine-2-carboxamide)éthyl]benzénesulfonyl]-(6-tert-pentyryloxy-3,4-dihydro-2,5,7,8-tétraméthyl-2H-1-benzopyrane-2-yl)-carboxamide

On procède comme dans la méthode 3 en utilisant le substrat de l'exemple 52 et le réactif de l'exemple 59.
Point de fusion : 85-90°C

### EXEMPLE 62 :

### 6-Acétoxy-3,4-dihydro-2,5,7,8-tétraméthyl-2H-1-benzopyrane-2-carboxylique acide{2-[4-(N'-[4-méthylcyclohexyl]-uréidosulfonyl-phényl]-méthyl}-amide

On procède comme dans la méthode 7 en utilisant comme réactif le 4-(2-aminométhyl)benzénesulfonamide, puis on traite selon la méthode 8 puis la méthode 9 en utilisant dans cette dernière étape le réactif utilisé dans l'exemple 38.
Point de fusion : 90°C

### EXEMPLE 63 :

### 4-Phtalimidométhyl-N-(6-acétoxy-3,4-dihydro-2,5,7,8-tétraméthyl-2H-1-benzopyrane-2-carbonyl)-benzènesulfonamide

On procède comme dans la méthode 3 en utilisant comme réactif le 4-Phtalimidométhyl-benzénesulfonamide.
Point de fusion : 90°C

### EXEMPLE 64 :

### 4-[2-Phtalimidoéthyl)-N-(6-acétoxy-3,4-dihydro-2,5,7,8-tétraméthyl-2H-1-benzopyrane-2-carbonyl)-benzènesulfonamide

On procède comme dans la méthode 3 en utilisant comme réactif le 4-(2-phtalimidoéthyl)-benzènesulfonamide
Point de fusion : 150°C

### ETUDE PHARMACOLOGIQUE

### A. RECHERCHE D'UNE ACTIVITE ANTIDIABETIQUE

### Animaux et traitements:

Des rats mâles Wistar d'environ 250 g et âgés de trois mois ont été utilisés pour toutes les expériences.

Les animaux sont maintenus dans une pièce à la température de 21 ± 2°C, avec alternance de périodes jour/nuit de durée de 12 heures. L'eau et la nourriture sont en libre accès.

Un diabète expérimental est obtenu par injection iv d'une faible dose de streptozotocine dissoute dans un tampon citrate sous anesthésie au chlorhydrate de kétamine (75 mg.kg-1, IP). Ces rats sont appelés "rats STZ". Les rats normaux ont reçu une injection de tampon citrate dans les mêmes conditions.

L'homéostasie glucidique a été évaluée par un test de tolérance au glucose, réalisé deux semaines après injection de streptozotocine.

### Test de tolérance au glucose :

◆ IVGTT (test de tolérance au glucose par voie intraveineuse)
   Le glucose est dissout dans une solution aqueuse de NaCI à 0,9 % et administré par la voie de la veine saphène à des rats anesthésiés au pentobarbital (60 mg.kg-1, lp). Les échantillons de sang sont collectés séquentiellement par les vaisseaux de la queue avant injection et 5, 10, 15, 20 et 30 minutes après injection du glucose. Ils sont ensuite centrifugés et le plasma est séparé. La concentration en glucose plasmatique est déterminée immédiatement sur un aliquot de 10 µl et le plasma restant est conservé à 20°C.
◆ OGTT (test de tolérance au glucose par voie orale)
   Le glucose est administré per os (2 g.kg-1) à des rats vigiles. Les échantillons de sang sont collectés avant et 10, 20, 30, 40, 90 et 120 minutes après administration du glucose. Le traitement des échantillons sanguins est identique à celui décrit précédemment.
◆ Administration des produits à tester
   Une injection unique en IP du produit à étudier est effectuée à des rats anesthésiés au pentobarbital, 20 minutes avant l'IVGTT. Le produit est administré per os 30 minutes avant l'OGTT.
◆ Produits utilisés
   - chlorhydrate de kétamine (Imalgène, Mérieux)
   - pentobarbital (Clin-Midy)

### Effet du produit sur la glycémie basale :

Une administration unique du produit à étudier, per os, est réalisée chez des rats en période post-prandiale. Les échantillons de sang sont collectés par les vaisseaux de la queue, avant et toutes les 30 minutes pendant 3 heures après administration du produit. Le taux de glucose plasmatique est mesuré immédiatement.

### Méthodes analytiques :

La concentration en glucose plasmatique est déterminée par utilisation d'un analyseur de glucose (Beckman Inc., Fullerton, CA). La tolérance au glucose est mesurée par rapport à deux paramètres : △G et K.

△G représente l'augmentation de la glycémie au dessus de la ligne de base, intégrée sur une base de 30 minutes (IVGTT) ou de 120 minutes (OGTT), après surcharge en glucose.

K est la vitesse de disparition du glucose entre 5 et 30 minutes (IVGTT), après administration du glucose. Le coefficient K est calculé uniquement pendant l'IVGTT.

Les produits de l'invention possèdent une bonne activité sur ces tests prédictifs d'une utilisation dans le traitement du diabète

### B. ETUDE DE L'ACTIVITE ANTIPEROXYDANTE :

L'action des composés de l'invention, susceptibles de piéger les radicaux OH a été étudiée d'une part, sur la peroxydation spontanée des lipides et d'autre part, sur la peroxydation induite par le système Fe2+-ascorbate (10 µM - 250µM) et ce sur des homogénats de cerveaux de rats.

Lors de la mesure de la peroxydation lipidique spontanée, les homogénats de cerveau de rats sont placés en présence ou en absence des composés à tester durant 60 minutes à 37°C. La réaction est arrêtée à 0°C et le dosage du malondialdéhyde est effectué à l'aide d'acide thiobarbiturique par la méthode de YAGI, K (1976) Biochem. Med, 15, 212 - 216. La peroxydation lipidique est déterminée par les substances réagissant avec l'acide thiobarbiturique exprimées en nanomoles de malondialdéhyde.

Lors de la mesure de la peroxydation lipidique induite, la méthodologie est identique à la précédente à l'exception de l'addition à l'homogénat du système inducteur de radicaux : Fe2+ - ascorbate. Les substances de référence sont le probucol et la vitamine E.

Les concentrations des composés testés inhibant de 50 % la peroxydation du substrat sont calculées.

Il est apparu que certains composés de l'invention possèdent une activité antiperoxydante particulièrement intense. Ce résultat très intéressant se produit que la peroxydation soit spontanée ou induite par un système chimique.

### C. ETUDE DU POUVOIR PROTECTEUR DE L'OXYDATION DES LDL :

La capacité des composé de l'invention à diminuer les proportions de LDL a été mesurée de la façon suivante. On réalise une incubation de 24 heures regroupant des LDL natives, un système Cu²⁺ générateur de radicaux libres et les composés à tester.

Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol.

Il apparaît clairement que les composés de l'invention ont un pouvoir protecteur très important et significativement supérieur à celui du composé de référence.

L'activité du produit de l'invention sur ces deux tests permet d'envisager également une activité intéressante sur les effets secondaires de la maladie diabétique.

### TOXICITE AIGUE

La toxicité aigue a été appréciée après administration orale à des lots de 8 souris (26 ± 2 grammes) d'une dose de 650 mg.kg⁻¹. Les animaux ont été observés à intervalles réguliers au cours de la première journée et quotidiennement pendant les deux semaines suivant le traitement.

Il apparaît que la plupart des composés de l'invention sont totalement atoxiques. La plupart d'entre eux n'entraîne aucun décès après administration à une dose de 650 mg.kg⁻¹ et on ne constate généralement pas de troubles après administration de cette dose.

### COMPOSITIONS PHARMACEUTIQUES :

Comprimés destinés au traitement du diabète ou des effets secondaires de la maladie diabétique dosés à 50 mg de 4-Méthyl-N-(6-acétoxy-3,4-dihydro-2,5,7,8-tétraméthyl-2H-1-benzopyrane-2-carbonyl)-benzènesulfonamide.

| Formule de préparation pour 1000 comprimés : | |
|---|---|
| 4-Méthyl-N-(6-acétoxy-3,4-dihydro-2,5,7,8-tétraméthyl-2H-1-benzopyrane-2-carbonyl)-benzènesulfonamide | 50 g |
| Amidon de blé | 50 g |
| Amidon de maïs | 50 g |
| Lactose | 175 g |
| Stéarate de magnésium | 5 g |
| Silice | 2,5 g |
| Hydroxypropylcellulose | 5 g |

## Revendications

1. Composés de formule (I) : 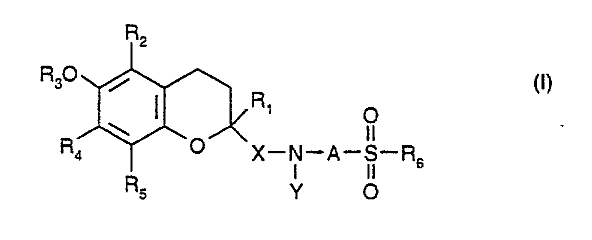 dans laquelle :
R₁ représente un groupement alkyle (C₁-C₉) linéaire ou ramifié ;
R₂, R₄, R₅ identiques ou différents, représentent chacun indépendamment l'un de l'autre, un atome d'hydrogène ou un groupement alkyle (C₁-C₉) linéaire ou ramifié ;
R₃ représente un atome d'hydrogène, un groupement alkyle (C₁-C₉) linéaire ou ramifié, un groupement acyle (C₁-C₉) linéaire ou ramifié, un groupement carboxyalkyle (C₁-C₉) linéaire ou ramifié, un groupement alkoxycarbonyle (C₁-C₉) linéaire ou ramifié, ou un groupement 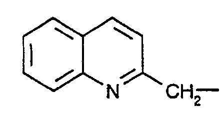 éventuellement substitué par un halogène en position 7 ;
X représente un groupement carbonyle ou un groupement méthylène ;
Y représente un atome d'hydrogène, un groupement alkyle (C₁-C₉) linéaire ou ramifié, ou un groupement aryle éventuellement substitué ;
A représente
• soit une liaison simple et alors:
R₆ représente un groupement alkyle (C₁-C₉) linéaire ou ramifié (éventuellement substitué par un groupement aryle lui-même éventuellement substitué), ou un groupement aryle éventuellement substitué ;
• soit un groupement alkylphényle (éventuellement substitué sur le phényle) -R_{b}-Ph-, la partie R_{b} alkyle (C₁ - C₉) linéaire ou ramifié étant rattachée au groupement 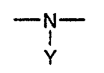 et alors :
R₆ représente :
• un groupement isocyanate,
• un groupement amino éventuellement substitué par un ou deux groupements alkyles (C₁-C₉) linéaires ou ramifiés, identiques ou différents, ou par un groupement alcoxycarbonyle (C₁-C₉) linéaire ou ramifié,
• ou un groupement urée substitué -NH-CO-NH-R₇ avec R₇ représentant un groupement phényle (éventuellement substitué), un groupement cycloalkyle (C₅-C₈) (éventuellement substitué par un groupement alkyle (C₁-C₉) linéaire ou ramifié), ou un groupement 3-azabicyclo [3.3.0]octyle,
par groupement aryle on entend un groupement phényle ou naphtyle et par groupement aryle substitué ou phényle substitué on entend une substitution par 1 à 3 groupements choisis identiques ou différents et indépendamment l'un de l'autre parmi :
- alkyle (C₁-C₉) linéaire ou ramifié,
- alkoxy (C₁-C₉) linéaire ou ramifié,
- alkoxycarbonyle (C₁-C₉) linéaire ou ramifié,
- carboxyle,
- carboxyalkyle (C₁-C₉) linéaire ou ramifié,
- acyle (C₁-C₉) linéaire ou ramifié,
- alkylcarbonylamino (C₁-C₉) linéaire ou ramifié,
- aminoalkyle (C₁-C₉) linéaire ou ramifié,
- 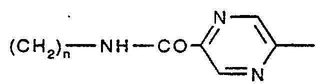 où n est compris entre 1 et 6 inclus,
- amino éventuellement substitué par un ou deux groupements alkyles (C₁ - C₉) linéaires ou ramifiés, identiques ou différents,
- alkényle (C₁-C₉) linéaire ou ramifié,
- nitro,
- halogéno,
- trihalogénométhyle,
- phtalimidoalkyle (C₁-C₉) linéaire ou ramifié,
leurs isomères, ainsi que leurs sels d'addition à un acide ou à une base pharmaceutiquement acceptable.

2. Composés de formule (I) selon la revendication 1 caractérisés en ce que :
R₁, R₂, R₄ et R₅, identiques ou différents, représentent un groupement alkyle (C₁-C₉) linéaire ou ramifié,
R₃ représente un atome d'hydrogène ou un groupement acyle (C₁-C₉) linéaire ou ramifié,
X représente un groupement carbonyle,
Y représente un atome d'hydrogène ou un groupement alkyle (C₁-C₉) linéaire ou ramifié et de façon préférentielle un atome d'hydrogène,
A représente
- une liaison simple et alors R₆ représente avantageusement un groupement aryle éventuellement substitué,
- ou un phényléthyle et alors R₆ représente avantageusement une urée substituée -NH-CO-NH-R₇ dans laquelle R₇ représente un groupement phényle (éventuellement substitué), un groupement cycloalkyle (C₅-C₈) éventuellement substitué par un groupement alkyle (C₁-C₉) linéaire ou ramifié, ou un groupement 3-azabicyclo[3.3.0]octyle,
leurs isomères ainsi que leurs sels d'addition à un acide ou à une base pharmaceutiquement acceptable

3. Composés de formule (I) selon l'une quelconque des revendications 1 et 2 caractérisés en ce que :
A représente une liaison simple et R₆ représente un groupement phényle substitué par un groupement alkyle (C₁-C₉) linéaire ou ramifié, un halogène ou un groupement 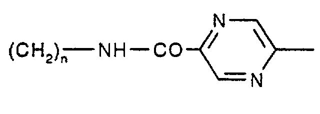 où n est compris entre 1 et 6 inclus, leurs isomères ainsi que leurs sels d'addition à un acide ou à une base pharmaceutiquement acceptable.

4. Composé de formule (I) selon la revendication 1 qui est le 4-Chloro-N-(6-acétoxy-3,4-dihydro-2,5,7,8-tétraméthyl-2H-1-benzopyrane-2-carbonyl)-benzènesulfonamide, et ses sels d'addition à un acide ou à une base pharmaceutiquement acceptable.

5. Composé de formule (I) selon la revendication 1 qui est le 4-Bromo-N-(6-acétoxy-3,4-dihydro-2,5,7,8-tétraméthyl-2H-1-benzopyrane-2-carbonyl)-benzènesulfonamide et ses sels d'addition à un acide ou à une base pharmaceutiquement acceptable.

6. Composé de formule (I) selon la revendication 1 qui est le 4-Méthyl-N-(6-acétoxy-3,4-dihydro-2,5,7,8-tétraméthyl-2H-1-benzopyrane-2-carbonyl)-benzènesulfonamide et ses sels d'addition à un acide ou à une base pharmaceutiquement acceptable.

7. Procédé de préparation des composés de formule (I) selon la revendication 1, caractérisé en ce que l'on utilise comme produit de départ, un composé de formule (II) : 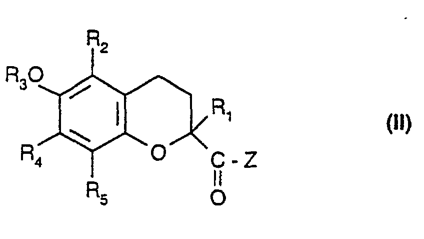 dans laquelle :
Z représente un groupement hydroxyle ou un atome d'halogène, et R₁, R₂, R₃, R₄ et R₅ ont la même signification que dans la formule (I),
que l'on condense :
• soit, lorsque A dans la formule (I) représente une liaison simple, avec un composé de formule (III) : 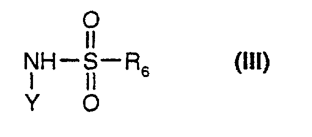 dans laquelle Y et R₆ ont la même signification que dans la formule (I),
pour conduire au composé de formule (l/a), cas particulier des composés de formule (I): 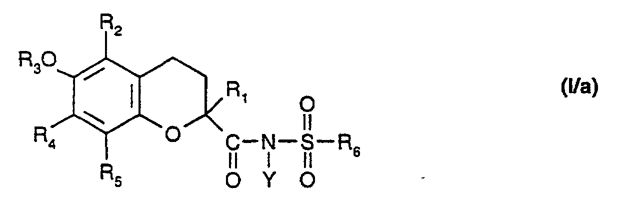 dans laquelle R₁, R₂, R₃, R₄, R₅, R₆ et Y ont la même signification que dans la formule (I), composé de formule (I/a) dont on réduit la fonction carbonyle, si on le souhaite, pour. conduire au composé de formule (I/b), cas particulier des composés de formule (I) : 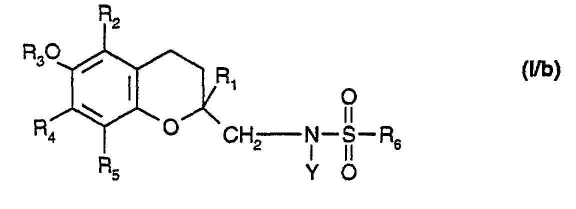 dans laquelle R₁, R₂, R₃, R₄, R₅, R₆, et Y ont la même signification que précédemment (I),
• soit, lorsque A dans la formule (I) représente un groupement alkylphényle -R_{b}-Ph-, avec un composé de formule (IV) : 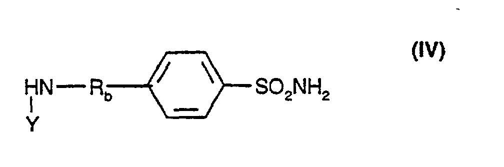 dans laquelle Y et R_{b} ont la même signification que dans la formule (I), pour conduire à un mélange de deux composés de formules respectives (l/c) et (l/d), cas particulier des composés de formule (I) 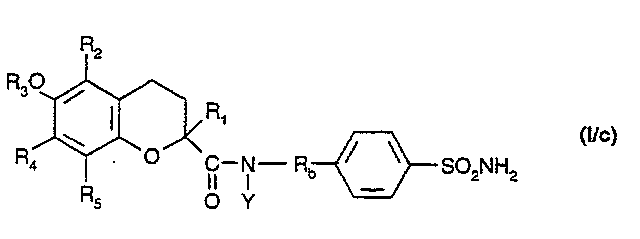 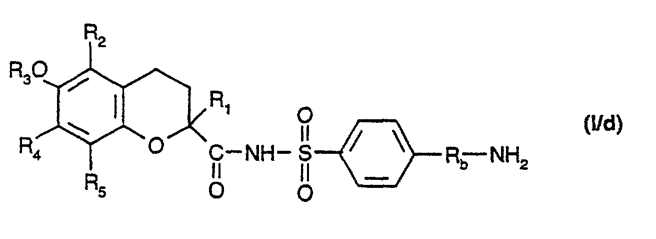 formules (I/c) et (I/d) dans lesquelles R_{b}, R₁, R₂, R₃, R₄, R₅ et Y ont la même signification que dans la formule (I), mélange dont on isole selon les procédés classiques de chromatographie, le composé de formule (I/c) que l'on traite par le phosgène pour conduire au composé de formule (I/e), cas particulier des composés de formule (I) : 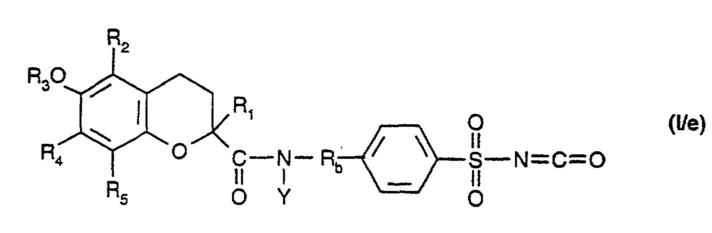 dans laquelle, R_{b}, R₁, R₂, R₃, R₄, R₅ et Y ont la même signification que dans la formule (I), composé de formule (I/e) que l'on traite par un composé de formule (V):
H₂N-R₇ (V)
dans laquelle R₇ a la même signification que dans la définition générale de la formule (I), pour donner des composés de formule (I/f), cas particulier des composés de formule (I) 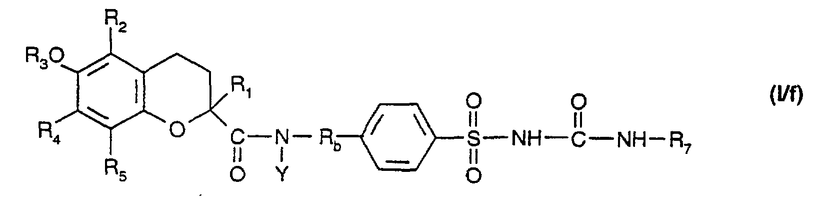 dans laquelle R_{b}, R₁, R₂, R₃, R₄, R₅, R₇ et Y ont la même signification que celle présentée dans la description générale de la formule (I),
composés de formules (I/a) à (I/f), que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

8. Compositions pharmaceutiques contenant comme principe actif au moins un composé selon l'une quelconque des revendications 1 à 6, seul ou en combinaison avec un ou plusieurs excipients ou véhicules inertes, non toxiques, pharmaceutiquement acceptables.

9. Compositions pharmaceutiques selon la revendication 8 contenant au moins un principe actif selon l'une quelconque des revendications 1 à 6 utiles dans le traitement du diabète et des complications de la maladie diabétique.

## Claims

1. Compounds of formula (I): 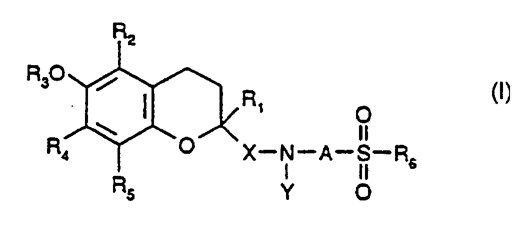 in which:
R₁ represents a linear or branched (C₁-C₉)alkyl group;
R₂, R₄ and R₅, which may be identical or different, each independently of the others represents a hydrogen atom or a linear or branched (C₁-C₉)alkyl group;
R₃ represents a hydrogen atom, a linear or branched (C₁-C₉)alkyl group, a linear or branched (C₁-C₉)acyl group, a carboxyalkyl group in which alkyl is linear or branched (C₁-C₉)alkyl, a linear or branched (C₁-C₉)alkoxycarbonyl group, or a 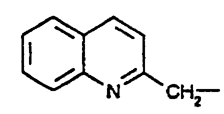 group optionally substituted by a halogen in the 7-position;
X represents a carbonyl group or a methylene group;
Y represents a hydrogen atom, a linear or branched (C₁-C₉)alkyl group, or an optionally substituted aryl group;
A represents
• either a single bond, and in that case:
R₆ represents a linear or branched (C₁-C₉)alkyl group (optionally substituted by an aryl group which is itself optionally substituted), or an optionally substituted aryl group;
• or an alkylphenyl group (optionally substituted at the phenyl) -R_{b}-Ph-, the R_{b} moiety being linear or branched (C₁-C₉)alkyl and being bonded to the 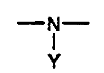 group, and in that case:
R₆ represents:
• an isocyanate group,
• an isocyanate group,
• an amino group optionally substituted by one or two identical or different linear or branched (C₁-C₉)alkyl groups, or by a linear or branched (C₁-C₉)-alkoxycarbonyl group,
• or a substituted urea group -NH-CO-NH-R₇ wherein R₇ represents a phenyl group (optionally substituted), a (C₅-C₈)cycloalkyl group (optionally substituted by a linear or branched (C₁-C₉)alkyl group), or a 3-azabicydo[3.3.0]-octyl group,
aryl group being understood as meaning a phenyl or naphthyl group, and substituted aryl group or substituted phenyl group being understood as meaning substitution by from 1 to 3 identical or different groups selected independently of one another from:
- linear or branched (C₁-C₉)alkyl,
- linear or branched (C₁-C₉)alkoxy,
- linear or branched (C₁-C₉)alkoxycarbonyl,
- carboxyl,
- carboxyalkyl in which alkyl is linear or branched (C₁-C₉)alkyl,
- linear or branched (C₁-C₉)acyl,
- linear or branched (C₁-C₉)alkylcarbonylamino,
- linear or branched (C₁-C₉)aminoalkyl,
- 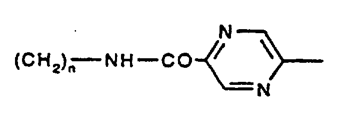 wherein n is from 1 to 6 inclusive,
- amino optionally substituted by one or two identical or different linear or branched (C₁-C₉)alkyl groups,
- linear or branched (C₁-C₉)alkenyl,
- nitro,
- halogen,
- trihalomethyl, and
- phthalimidoalkyl in which alkyl is linear or branched (C₁-C₉)alkyl,

2. Compounds of formula (I) according to claim 1, characterised in that:
R₁, R₂, R₄ and R₅, which may be identical or different, each represents a linear or branched (C₁-C₉)alkyl group,
R₃ represents a hydrogen atom or a linear or branched (C₁-C₉)acyl group,
X represents a carbonyl group,
Y represents a hydrogen atom or a linear or branched (C₁-C₉)alkyl group, preferably a hydrogen atom,
A represents
- a single bond, and in that case R₆ advantageously represents an optionally substituted aryl group,
- or a phenylethyl, and in that case R₆ advantageously represents a substituted urea -NH-CO-NH-R₇ wherein R₇ represents a phenyl group (optionally substituted), a (C₅-C₈)cycloalkyl group optionally substituted by a linear or branched (C₁-C₉)alkyl group, or a 3-azabicyclo[3.3.0]octyl group,
their isomers, and addition salts thereof with a pharmaceutically acceptable acid or base.

3. Compounds of formula (I) according to any one of claims 1 and 2, characterised in that:
A represents a single bond and R₆ represents a phenyl group substituted by a linear or branched (C₁-C₉)alkyl group, a halogen or by a group 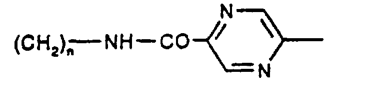 wherein n is from 1 to 6 inclusive,
their isomers, and addition salts thereof with a pharmaceutically acceptable acid or base.

4. Compound of formula (I) according to claim 1 which is 4-chloro-N-(6-acetoxy-3,4-dihydro-2,5,7,8-tetramethyl-2H-1-benzopyran-2-carbonyl)-benzenesulfonamide, and its addition salts with a pharmaceutically acceptable acid or base.

5. Compound of formula (I) according to claim 1 which is 4-bromo-N-(6-acetoxy-3,4-dihydro-2,5,7,8-tetramethyl-2H-1-benzopyran-2-carbonyl)-benzenesulfonamide, and its addition salts with a pharmaceutically acceptable acid or base.

6. Compound of formula (I) according to claim 1 which is 4-methyl-N-(6-acetoxy-3,4-dihydro-2,5,7,8-tetramethyl-2H-1-benzopyran-2-carbonyl)-benzenesulfonamide, and its addition salts with a pharmaceutically acceptable acid or base.

7. Process for the preparation of compounds of formula (I) according to claim 1, characterised in that there is used as starting material a compound of formula (II): 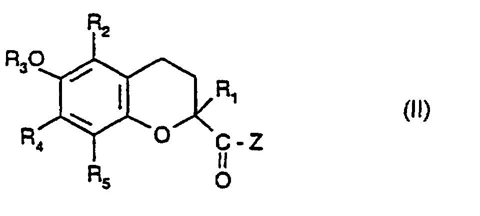 in which Z represents a hydroxyl group or a halogen atom, and R₁, R₂, R₃, R₄ and R₅ are as defined for formula (I),
which is condensed:
• either, when A in formula (I) represents a single bond, with a compound of formula (III): 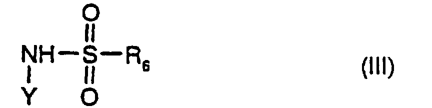 in which Y and R₆ are as defined for formula (I),
to yield a compound of formula (I/a), a particular case of the compounds of formula (I): 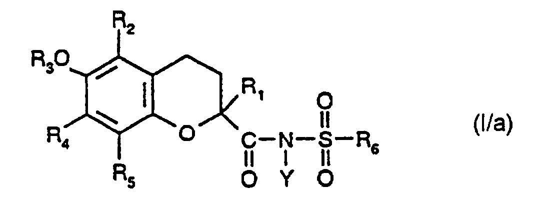 in which R₁, R₂, R₃, R₄, R₅, R₆ and Y are as defined for formula (I), the carbonyl function of which compound of formula (l/a) is reduced, if desired, to yield a compound of formula (l/b), a particular case of the compounds of formula (I): 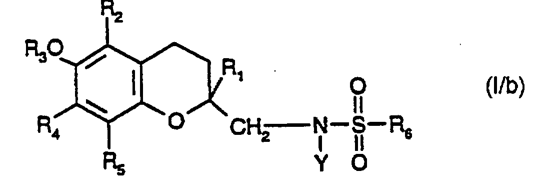 in which R₁, R₂, R₃, R₄, R₅, R₆ and Y are as defined for formula (I),
• or, when A in formula (I) represents an alkylphenyl group -R_{b}-Ph-, with a compound of formula (IV): 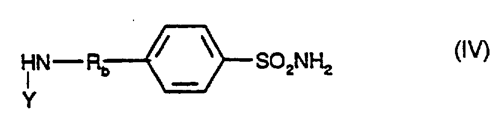 in which Y and R_{b} are as defined for formula (I), to yield a mixture of two compounds of formulae (I/c) and (I/d), particular cases of the compounds of formula (I) 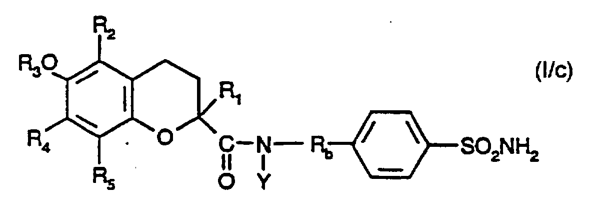 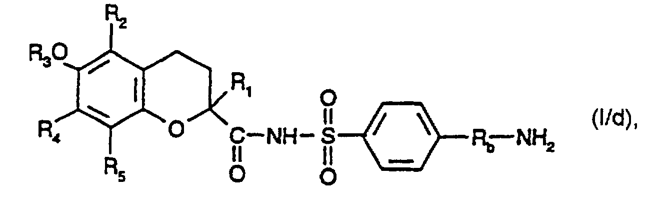 in which formulae (I/c) and (I/d) R_{b}, R₁, R₂, R₃, R₄, R₅ and Y are as defined for formula (I), from which mixture the compound of formula (I/c) is isolated by conventional chromatographic processes and is treated with phosgene to yield a compound of formula (l/e), a particular case of the compounds of formula (I): 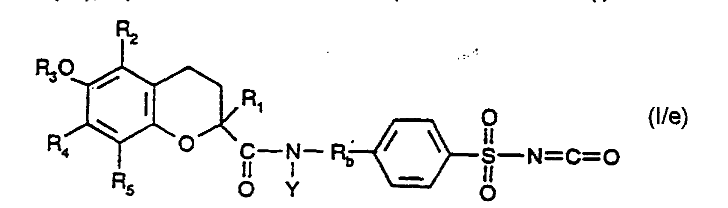 in which R_{b}, R₁, R₂, R₃, R₄, R₅ and Y are as defined for formula (I), which compound of formula (l/e) is treated with a compound of formula (V):
H₂N - R₇ (V)
in which R₇ is as defined in the general definition of formula (I), to yield compounds of formula (l/f), a particular case of the compounds of formula (I) 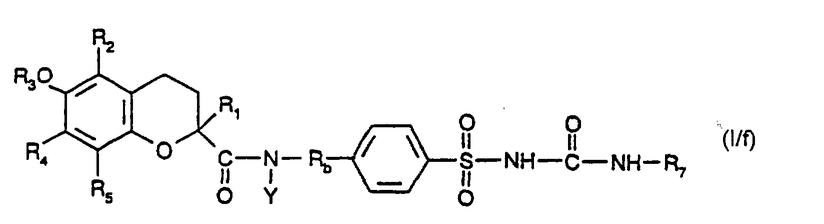 in which R_{b}, R₁, R₂, R₃, R₄, R₅, R₇ and Y are as defined in the general description of formula (1),
which compounds of formulae (l/a) to (l/f) are purified, where appropriate, by a conventional purification technique, are separated into their isomers, where appropriate, by a conventional separation technique, and are converted, if desired, into addition salts with a pharmaceutically acceptable acid or base.

8. Pharmaceutical compositions comprising as active ingredient at least one compound according to any one of claims 1 to 6, alone or in combination with one or more inert, non-toxic, pharmaceutically acceptable excipients or carriers.

9. Pharmaceutical compositions according to claim 8 comprising at least one active ingredient according to any one of claims 1 to 6, for use in the treatment of diabetes and of complications of diabetic illness.

## Patentansprüche

1. Verbindungen der Formel (I): 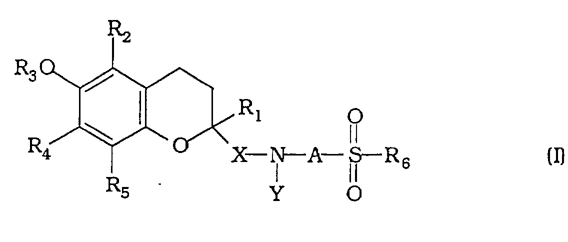 in der:
R₁ eine geradkettige oder verzweigte (C₁-C₉)-Alkylgruppe;
R₂, R₄ und R₅, die gleichartig oder verschieden sind, jeweils unabhängig voneinander ein Wasserstoffatom oder eine geradkettige oder verzweigte (C₁-C₉)-Alkylgruppe;
R₃ ein Wasserstoffatom, eine geradkettige oder verzweigte (C₁-C₉)-Alkylgruppe, eine geradkettige oder verzweigte (C₁-C₉)-Acylgruppe, eine geradkettige oder verzweigte (C₁-C₉)-Carboxyalkylgruppe, eine geradkettige oder verzweigte (C₁-C₉)-Alkoxycarbonylgruppe oder eine Gruppe der Formel 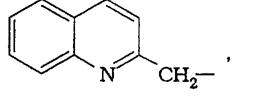 die gegebenenfalls in der 7-Stellung durch Halogen substituiert ist;
X eine Carbonylgruppe oder eine Methylengruppe;
Y ein Wasserstoffatom, eine geradkettige oder verzweigte (C₁-C₉)-Alkylgruppe oder eine gegebenenfalls substituierte Arylgruppe;
A
• entweder eine Einfachbindung und in diesem Fall:
R₆ eine geradkettige oder verzweigte (C₁-C₉)-Alkylgruppe (die gegebenenfalls durch eine ihrerseits gegebenenfalls substituierte Arylgruppe substituiert ist) oder eine gegebenenfalls substituierte Arylgruppe darstellt;
• oder eine (gegebenenfalls am Phenylrest substituierte) Alkylphenylgruppe -R_{b}-Ph-, wobei der geradkettige oder verzweigte (C₁-C₉)-Alkylteil R_{b} an die Gruppe 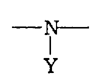 gebunden ist und in diesem Fall:
R₆:
• eine Isocyanatgruppe,
• eine Aminogruppe, die gegebenenfalls durch eine oder zwei gleichartige oder verschiedene geradkettige oder verzweigte (C₁-C₉)-Alkylgruppen oder durch eine geradkettige oder verzweigte (C₁-C₉)-Alkoxycarbonylgruppe substituiert ist,
• oder eine substituierte Harnstoffgruppe -NH-CO-NH-R₇ darstellt, worin R₇ eine (gegebenenfalls substituierte) Phenylgruppe, eine (gegebenenfalls durch eine geradkettige oder verzweigte (C₁-C₉)-Alkylgruppe substituierte) (C₅-C₈)-Cycloalkylgruppe oder eine 3-Azabicyclo[3.3.0]octylgruppe darstellt, bedeuten,
wobei unter einer Arylgruppe eine Phenyl- oder Naphthylgruppe und unter einer substituierten Arylgruppe oder substituierten Phenylgruppe eine Substitution mit 1 bis 3 gleichartigen oder verschiedenen Gruppen zu verstehen ist, die unabhängig voneinander ausgewählt sind aus:
- geradkettigem oder verzweigtem (C₁-C₉)-Alkyl,
- geradkettigem oder verzweigtem (C₁-C₉)-Alkoxy,
- geradkettigem oder verzweigtem (C₁-C₉)-Alkoxycarbonyl,
- Carboxyl,
- geradkettigem oder verzweigtem (C₁-C₉)-Carboxyalkyl,
- geradkettigem oder verzweigtem (C₁-C₉)-Acyl,
- geradkettigem oder verzweigtem (C₁-C₉)-Alkylcarbonylamino,
- geradkettigem oder verzweigtem (C₁-C₉)-Aminoalkyl,
- 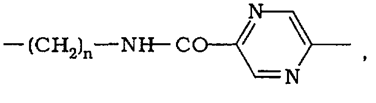 worin n zwischen 1 und 6 einschließlich liegt,
- gegebenenfalls substituiert durch ein oder zwei gleichartige oder verschiedene, geradkettige oder verzweigte (C₁-C₉)-Alkylgruppen,
- geradkettigem oder verzweigtem (C₁-C₉)-Alkenyl,
- Nitro,
- Halogen,
- Trihalogenmethyl und
- geradkettigem oder verzweigtem Phthalimido-(C₁-C₉)-alkyl,
deren Isomere sowie deren Additionssalze mit einer pharmazeutisch annehmbaren Säure oder Base.

2. Verbindungen der Formel (I) nach Anspruch 1, dadurch gekennzeichnet, daß:
R₁, R₂, R₄ und R₅, die gleichartig oder verschieden sind, geradkettige oder verzweigte (C₁-C₉)-Alkylgruppen,
R₃ ein Wasserstoffatom oder eine geradkettige oder verzweigte (C₁-C₉)-Alkylgrup-pe,
X eine Carbonylgruppe,
Y ein Wasserstoffatom oder eine geradkettige oder verzweigte (C₁-C₉)-Alkylgruppe, vorzugsweise ein Wasserstoffatom, und
A
- eine Einfachbindung und in diesem Fall R₆ mit Vorteil eine gegebenenfalls substituierte Arylgruppe,
- oder Phenylethyl und in diesem Fall R6 mit Vorteil eine substituierte Harnstoffgruppe -NH-CO-NH-R₇, worin R₇ eine (gegebenenfalls substituierte) Phenylgruppe, eine (C₅-C₈)-Cycloalkylgruppe, die gegebenenfalls durch eine geradkettige oder verzweigte (C₁-C₉)-Alkylgruppe substituiert ist, oder eine 3-Azabicyclo[3.3.0]octylgruppe darstellt, bedeuten,
deren Isomere sowie deren Additionssalze mit einer pharmazeutisch annehmbaren Säure oder Base.

3. Verbindungen der Formel (I) nach einem der Ansprüche 1 und 2, dadurch gekennzeichnet, daß:
A eine Einfachbindung und R₆ eine Phenylgruppe, die durch eine geradkettige oder verzweigte (C₁-C₉)-Alkylgruppe, ein Halogen oder eine Gruppe 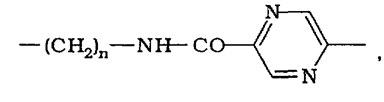 worin n zwischen 1 und 6 einschließlich liegt, substituiert ist, bedeuten,
deren Isomere sowie deren Additionssalze mit einer pharmazeutisch annehmbaren Säure oder Base.

4. Verbindung der Formel (I) nach Anspruch 1, nämlich 4-Chlor-N-(6-acetoxy-3,4-dihydro-2,5,7,8-tetramethyl-2H-1-benzopyran-2-carbonyl)-benzolsulfonamid und dessen Additionssalze mit einer pharmazeutisch annehmaren Säure oder Base.

5. Verbindung der Formel (I) nach Anspruch 1, nämlich 4-Brom-N-(6-acetoxy-3,4-dihydro-2,5,7,8-tetramethyl-2H-1-benzopyran-2-carbonyl)-benzolsulfonamid und dessen Additionssalze mit einer pharmazeutisch annehmbaren Säure oder Base.

6. Verbindung der Formel (I) nach Anspruch 1, nämlich 4-Methyl-N-(6-acetoxy-3,4-dihydro-2,5,7,8-tetramethyl-2H-1-benzopyran-2-carbonyl)-benzolsulfonamid und dessen Additionssalze mit einer pharmazeutisch annehmbaren Säure oder Base.

7. Verfahren zur Herstellung der Verbindungen der Formel (I) nach Anspruch 1, dadurch gekennzeichnet, daß man als Ausgangsprodukt eine Verbindung der Formel (II) verwendet: 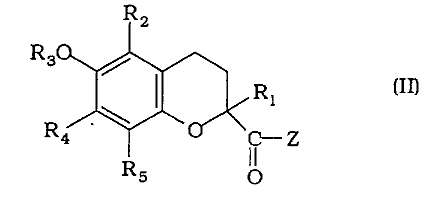 in der:
Z eine Hydroxygruppe oder ein Halogenatom bedeutet und R₁, R₂, R₃, R₄ und
R₅ die bezüglich der Formel (I) angegebenen Bedeutungen besitzen, welches man:
• entweder, wenn A der Formel (I) eine Einfachbindung darstellt, mit einer Verbindung der Formel (III) kondensiert: 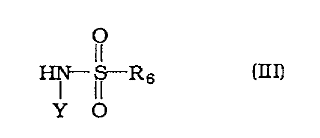 in der Y und R₆ die bezüglich der Formel (I) angegebenen Bedeutungen besitzen,
zur Bildung der Verbindung der Formel (I/a), einem Sonderfall der Verbindungen der Formel (I): 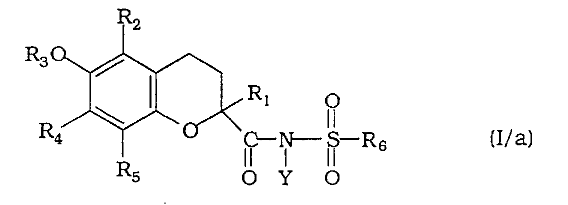 in der R₁, R₂, R₃, R₄, R₅, R₆ und Y die bezüglich der Formel (I) angegebenen Bedeutungen besitzen,
deren Carbonylfunktion der Verbindung der Formel (I/a) man gewünschtenfalls reduziert zur Bildung der Verbindung der Formel (I/b), einem Sonderfall der Verbindungen der Formel (I): 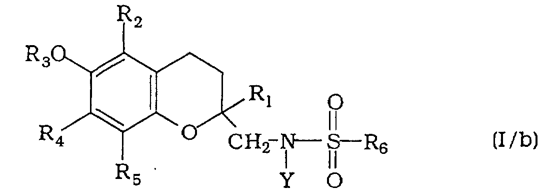 in der R₁, R₂, R₃, R₄, R₅, R₆ und Y die bezüglich der Formel (I) angegebenen Bedeutungen besitzen,
• oder, wenn A der Formel (I) eine Alkylphenylgruppe -R_{b}-Ph- darstellt, mit einer Verbindung der Formel (IV) kondensiert: 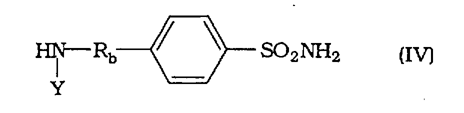 in der Y und R_{b} die bezüglich der Formel (I) angegebenen Bedeutungen besitzen, zur Bildung einer Mischung der beiden jeweiligen Verbindungen der Formeln (I/c) und (I/d), Sonderfällen der Verbindungen der Formel (I): 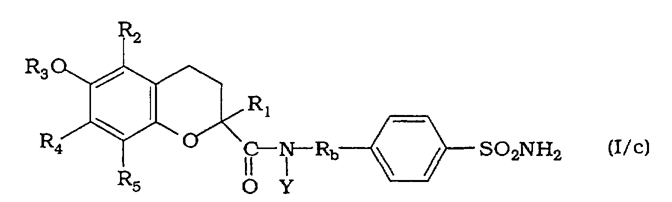 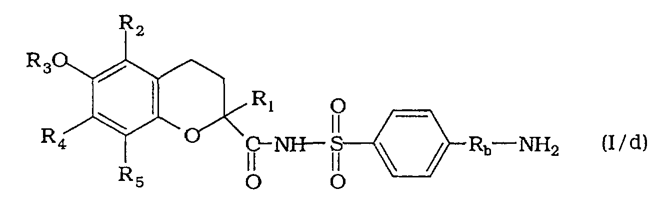 in welchen Formeln (I/c) und (I/d) R_{b}, R₁, R₂, R₃, R₄, R₅ und Y die bezüglich der Formel (I) angegebenen Bedeutungen besitzen,
aus welcher Mischung man mit Hilfe klassischer Chromatographieverfahren die Verbindung der Formel (I/c) isoliert und mit Phosgen behandelt zur Bildung der Verbindung der Formel (I/e), einem Sonderfall der Verbindungen der Formel (I): 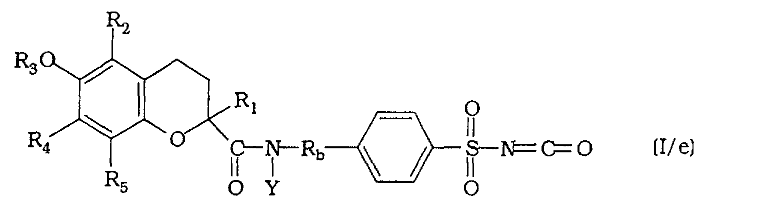 in der R_{b}, R₁, R₂, R₃, R₄, R₅ und Y die bezüglich der Formel (I) angegebenen Bedeutungen besitzen,
welche Verbindung der Formel (I/e) man mit einer Verbindung der Formel (V) behandelt:
H₂N - R₇ (V)
in der R₇ die bezüglich der allgemeinen Formel (I) angegebenen Bedeutungen besitzt,
zur Bildung der Verbindungen der Formel (I/f), einem Sonderfall der Verbindungen der Formel(I): 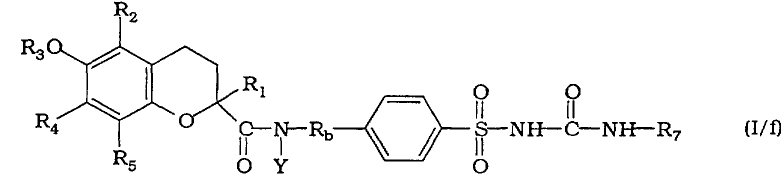 worin R_{b}, R₁, R₂, R₃, R₄, R₅, R₇ und Y die bezüglich der allgemeinen Beschreibung der Formel (I) angegebenen Bedeutungen besitzen,
welche Verbindungen der Formeln (I/a) bis (I/f) man gegebenenfalls mit Hilfe einer klassischen Reinigungsmethode reinigt, gegebenenfalls mit Hilfe einer klassischen Trennmethode in ihre Isomeren auftrennt und gewünschtenfalls in ihre Additionssalze mit einer pharmazeutisch annehmbaren Säure oder Base überführt.

8. Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien oder Bindemitteln.

9. Pharmazeutische Zubereitungen nach Anspruch 8 enthaltend mindestens einen Wirkstoff nach einem der Ansprüche 1 bis 6 zur Behandlung des Diabetes und von Komplikationen von Diabetes-Erkrankungen.
